# EUROPÄISCHE PATENTSCHRIFT

(11) **EP 1 639 998 B1**
(45) Veröffentlichungstag und Bekanntmachung des Hinweises auf die Patenterteilung: **10.10.2007**
(21) Anmeldenummer: 05016940.8
(22) Anmeldetag: 04.08.2005
(51) Int. Cl.: A61K 9/20, A61K 36/09, A61K 36/00, A61P 11/04

(54) **Schleimdrogenbasierte Lutschtablette gegen entzündliche Erkrankungen des Mund- und Rachenraums**
Plant mucilage based lozenges against inflammatory diseases of the buccal and pharyngeal cavity
Losange à base de mucilage de plantes contre les processus inflammatoires de la cavité buccale et pharyngienne

(30) Priorität: 22.09.2004 DE 102004046267; 28.09.2004 DE 102004047405; 16.10.2004 DE 102004050591
(43) Veröffentlichungstag der Anmeldung: 29.03.2006
(73) Patentinhaber: Maria Clementine Martin Klosterfrau Vertriebsgesellschaft mbH, 50670 Köln (DE)
(72) Erfinder: Vestweber, Anna-Maria, Dr. med., 51399 Burscheid (DE); Greve, Harald, Dr., 40541 Düsseldorf (DE)
(74) Vertreter: Gesthuysen, von Rohr & Eggert

(56) Entgegenhaltungen:
- EP-A- 0 686 351
- US-A- 3 341 414
- US-A- 5 713 852
- US-A1- 2003 003 140
- US-A1- 2004 136 923
- "An ancient but still valid medicament, Iceland moss" THERAPIEWOCHE 1982 GERMANY, Bd. 32, Nr. 16, 1982, Seite 2131, XP009058349

## Beschreibung

Die vorliegende Erfindung betrifft eine pharmazeutische Zusammensetzung in Form einer festen Dosierungsform, insbesondere auf Lutschtablettenbasis, welche eine Kombination von mindestens einer Schleimdroge in Form von deren Extrakt, insbesondere Isländisches Moos *(Lichen islandicus)* und/oder Eibisch *(Althaea officinalis L.),* zusammen mit mindestens einem Lokalanästhetikum auf Basis von Benzocain enthält.

Des weiteren betrifft die vorliegende Erfindung die Verwendung der vorgenannten pharmazeutischen Zusammensetzung bzw. einer Kombination von mindestens einer Schleimdroge in Form von deren Extrakt zusammen mit mindestens einem Lokalanästhetikum auf Basis von Benzocain zur topischen Behandlung von insbesondere schmerzbegleiteten entzündlichen Erkrankungen des Mund- und Rachenraums.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf. Solche entzündlichen Prozesse des Mund- und Rachenraums sind oftmals mit einer für den betroffenen Patienten unangenehmen Schmerzsymptomatik verbunden. Nicht beschränkende Beispiele für derartige entzündliche Erkrankungen des Hals-/Rachenraums sind Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Solche Erkrankungen des Hals-/Rachenraums sind oftmals von schmerzhaften Schluckbeschwerden begleitet. Auch Entzündungen im Bereich der Mundhöhle, wie z. B. Stomatitis, Gingivitis, Mundschleimhautläsionen und dergleichen, sind oftmals von Schmerzen begleitet. Für weitere diesbezügliche Einzelheiten zu den vorgenannten Erkrankungen kann beispielsweise auf Roche-Lexikon Medizin, 3. Auflage, 1993, Urban & Schwarzenberg, München/Wien/Baltimore, sowie auf Pschyrembel, Medizinisches Wörterbuch, 257. Auflage, 1993, Nikol Verlagsgesellschaft mbH, Hamburg, verwiesen werden. Der Begriff "entzündliche Erkrankungen des Mund- und Rachenraums" ist somit sehr weit zu verstehen und umfaßt sämtliche entzündlichen Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im allgemeinen durch topische und gegebenenfalls zusätzliche systemische Therapie in schwerwiegenderen Fällen. Während in schwereren Fällen systemisch Antibiotika verabreicht werden, kommen unterstützend und in leichteren Fällen unter Umständen alleinig zur topischen, symptomatischen Behandlung oftmals Antiseptika und entzündungshemmende Stoffe (so z. B. Antiphlogistika, antiinflammatorisch wirkende Mittel, Lokalantibiotika etc.) zum Einsatz, welche beispielsweise in Form von Rachenspülungen, Sprays oder Lutschtabletten verabreicht werden können.

Als Wirkstoff zur topischen Behandlung von entzündlichen Prozessen des Mund- und Rachenraums hat sich insbesondere 1-Hexadecylpyridiniumchlorid (internationaler Freiname: "Cetylpyridiniumchlorid" (CPC)) bewährt. Hierbei handelt es sich um eine quaternäre Ammoniumverbindung mit bakterizider und fungizider Wirkung, welche unter anderem in Lutschtabletten zur Anwendung kommt. Nachteilig bei diesem Wirkstoff ist die Tatsache, daß bei hoher Dosierung und übermäßigem Verzehr Magen-Darm-Beschwerden, Atemnot sowie eine vermehrte Methämoglobinbildung, insbesondere bei Kindern, auftreten kann.

Des weiteren hat sich als topisches Antiseptikum bzw. Desinfektionsmittel für die Mund-, Hals- und Rachenschleimhaut auch 1,3-Bis(2-ethylhexyl)-hexahydro-5-methyl-5-pyridinamin (internationaler Freiname: "Hexetidin") bewährt, welches beispielsweise als Spray oder in Form von Spül- bzw. Gurgellösungen appliziert werden kann. Bei längerer Anwendung und starker Dosierung kann es auch hier zu Magen-Darm-Beschwerden und darüber hinaus zu Geschmacksirritationen kommen.

Des weiteren kommen Wirkstoffe natürlichen Ursprungs zum Einsatz, so z. B. sogenannte Schleimdrogen oder deren Extrakte, wie z. B. Isländisches Moos *(Lichen islandicus).* Derartige Präparate bieten aber nicht immer den gewünschten Therapieerfolg. Insbesondere berücksichtigen diese Präparate im allgemeinen nicht die mit den entzündlichen Erkrankungen des Mund- und Rachenraums einhergehende Schmerzsymptomatik.

Die US 3 341 414 A der Merck & Co., Inc. beschreibt Lutschtabletten auf Basis eines Antiphlogistikums in Kombination mit einem Lokalanästhetikum, wobei als Antiphlogistikum ausschließlich synthetische Stoffe, nämlich N-Cyclohexylsulfamate, zur Anwendung kommen.

Die US 200310003140 A1 beschreibt Tabletten mit pflanzlichen Extrakten mit entzündungshemmender Wirkung (z. B, *Echinacea)* in Kombination mit einem Lokalanästhetikum (z. B. Benzocain). Schleimdrogen werden in dieser Druckschrift nicht genannt.

Die US 5 713 852 A der Alza Corporation betrifft ein Verfahren zur Behandlung schmerzbegleiteter Entzündungen des Mund- und Rachenraums durch topische Behandlung mit einem Antiphlogistikum in Kombination mit einem Lokalanästhetikum, wobei ausschließlich synthetische Antiphlogistika zur Anwendung gelangen.

Die US 2004/0136923 A1 beschreibt einen eßbaren Film *("edible film")* zur Behandlung von Pharyngitis oder Husten mit einem Filmbildner und einer Wirksubstanz.

In der Druckschrift Therapiewoche 1982, Deutschland, Band 32, Nr. 16 (1982), Seite 2131, mit dem Titel "Ein Heilmittel in der Antike - heute noch aktuell / Lichen islandicus - bei entzündlichen Reizzuständen der Atemwege" ist die Wirksamkeit von *Lichen islandicus* bei entzündlichen Reizzuständen der Atemwege beschrieben, wobei diesbezüglich das Handelspräparat Isla Moos^{®} genannt wird, welches als einzigen Wirkstoff einen Extrakt von Isländischem Moos *(Lichen islandicus)* mit einem Droge/Extrakt-Verhältnis von 0,4 ; 1 bis 0,8 : 1 enthält. Eine Kombination mit einem Lokalanästhetikum wird nicht beschrieben.

Weiterhin beschreibt die EP 0 686 351 A2 ein Karamel und ein Verfahren zu seiner Herstellung, wobei in einer bevorzugten Ausführungsform der Karamel ein wäßriges Kräuterextrakt, organische Säuren und karamelisierten Zucker enthält, wobei als Basis für den Kräuterextrakt insbesondere Blüte oder Frucht des Holunders, Blüten der Königskerze, Lindenblüten, die Blüten des Kettenlattichkrauts und das Blattwerk des Salbeis in Betracht zu ziehen sind. Die Kombination einer Schleimdroge mit einem Lokalanästhetikum wird nicht genannt.

Der vorliegenden Erfindung liegt daher die Aufgabe zugrunde, eine pharmazeutische Zusammensetzung bereitzustellen, welche sich zur effizienten topischen Behandlung von insbesondere schmerzbegleiteten entzündlichen Erkrankungen des Mund- und Rachenraums (d. h. also von entzündlichen Erkrankungen des Hals-/Rachenraums und der Mundhöhle) eignet und insbesondere die zuvor geschilderten Nachteile des Standes der Technik zumindest weitgehend vermeidet oder wenigstens abschwächt.

Die Anmelderin hat nun überraschenderweise herausgefunden, daß das zuvor geschilderte Problem dadurch gelöst werden kann, daß man eine pharmazeutische Zubereitung in Form einer festen Dosierung auf Basis von mindestens einer Schleimdroge in Form von deren Extrakt (z. B. Isländisches Moos *(Lichen islandicus)* oder Eibisch (*Althaea officinalis L.*)) zusammen mit mindestens einem zur topischen Anwendung geeigneten Lokalanästhetikum auf Basis von Benzocain formuliert. Eine solche pharmazeutische Zusammensetzung eignet sich insbesondere zur Herstellung von Lutschtabletten, insbesondere auf Hartkaramellenbasis, welche bei schmerzhaften bzw. schmerzbegleiteten entzündlichen Erkrankungen des Mund- und Rachenraums zur topischen Anwendung gelangen können.

Die vorliegende Erfindung betrifft somit eine pharmazeutische Zusammensetzung nach Anspruch 1, Weitere vorteilhafte Ausgestaltungen sind Gegenstand der diesbezüglichen Unteransprüche.

Weiterhin betrifft die vorliegende Erfindung die Verwendung der erfindungsgemäßen pharmazeutischen Zusammensetzung nach Anspruch 8.

Gegenstand der vorliegenden Erfindung ist somit eine pharmazeutische Zusammensetzung in Form einer festen Dosierung zur topischen Behandlung von entzündlichen Erkrankungen des Mund- und Rachenraums, welche in Kombination
- mindestens eine Schleimdroge, ausgewählt aus Isländischem Moos *(Lichen islandicus),* Eibisch *(Althaea officinalis L.),* Spitzwegerich *(Plantago lanceolata L.),* Malve *(Malva sylvestris L.* und *M. neglecta WALLR.),* Boxhomklee *(Trigonella foenum-graecum L.),* Salep und Quitte *(Cydonia oblonga MILL.*), wobei die Schleimdroge in Form eines Extraktes mit einem Droge/Extrakt-Verhältnis im Bereich von 5 : 1 bis 8 : 1 in einer Menge von 0,5 bis 20 Gew.-%, bezogen auf die pharmazeutische Zusammensetzung, zugesetzt ist; und
- 0,05 bis 2 Gew.-%, bezogen auf die pharmazeutische Zusammensetzung, mindestens eines Lokalanästhetikums, wobei als Lokalanästhetikum Benzocain verwendet wird,
enthält.

Die vorgenannte Kombination eignet sich insbesondere zur temporären alleinigen oder unterstützenden Behandlung bei schmerzhaften Entzündungen des Mund- und Rachenraums, da die Kombination einerseits entzündungshemmend bzw. entzündungslindernd wirkt und zum anderen eine effiziente Schmerzlinderung bewirkt. Durch die Verwendung eines natürlich basierten Wirkstoffes auf Basis von mindestens einer Schleimdroge in Form von deren Extrakt (z. B. Isländisches Moos oder Eibisch) werden die mit synthetischen Wirkstoffen, wie z. B. Cetylpyridiniumchlorid und Hexetidin, auftretenden Nebenwirkungen wirksam verhindert.

Der Begriff "pharmazeutische Zusammensetzung" bzw. "pharmazeutische Kombination" - im Rahmen der vorliegenden Erfindung meist synonym verwendet - ist sehr weit zu verstehen und umfaßt jede Art von möglicher pharmazeutische Zusammensetzung oder Kombination, insbesondere Arzneimittel bzw. Pharmaka als solche, aber auch Medizinprodukte, homöopathische Mittel etc.

Die erfindungsgemäße pharmazeutische Zusammensetzung kann beispielsweise bei gereizter Schleimhaut mit Halsschmerzen oder Halsschmerzen bei ausgetrockneter Schleimhaut oder bei trockener Rachenschleimhaut mit Halsschmerzen eingesetzt werden. Darüber hinaus kann die erfindungsgemäße pharmazeutische Zusammensetzung bzw. Kombination gegen Halsschmerzen mit gleichzeitigem Schleimhautschutz eingesetzt werden. Durch die erfindungsgemäße Kombination wird eine effiziente Doppelwirkung erreicht: Einerseits wird eine effiziente Wirksamkeit gegen Halsschmerzen als solche bewirkt, und andererseits wird die Schleimhaut ausreichend benetzt, so daß einem Trockenheitsgefühl und einer Ausbreitung von Bakterien entgegengewirkt wird. Somit wirkt die erfindungsgemäße pharmazeutische Zusammensetzung bzw. Kombination aktiv gegen Halsschmerzen und Mundtrockenheit und bekämpft gleichermaßen die Entzündung. Somit eignet sich die erfindungsgemäße pharmazeutische Zusammensetzung beispielsweise für die unterstützende oder alleinige Behandlung bei Schleimhautreizung mit Halsschmerzen. Auf diese Weise werden Halsschmerzen effizient unter zwei Gesichtspunkten bekämpft, nämlich einerseits in bezug auf die Entzündung und andererseits in bezug auf die Schmerzsymptomatik. Die Benetzung der ausgetrockneten Mundschleimhaut bewirkt zudem, daß das Eindringen von weiteren Erregern erschwert wird. Die Regeneration der entzündlich veränderten Mundschleimhaut kann in den oberflächenanästhesierten Bereichen schmerzlos abheilen. Somit kommen zwei effiziente Wirkeffekte zum Tragen, nämlich zum einen der oberflächenanästhesierende Effekt und zum anderen der entzündungslindemde Effekt.

Die Anmelderin hat überraschenderweise herausgefunden, daß die erfindungsgemäße Kombination von mindestens einer Schleimdroge in Form von deren Extrakt (z. B. Isländisches Moos und/oder Eibisch) zusammen mit mindestens einem Lokalanästhetikum (nämlich Benzocain) die entzündungsbedingte Reizung der Mund- und Rachenschleimhaut deutlich mindert, und zwar über die Wirkung der jeweiligen einzelnen Stoffe hinausgehend. Dies deutet auf einen synergistischen Effekt in bezug auf die erfindungsgemäße Kombination hin.

Im Rahmen der vorliegenden Erfindung können eine Vielzahl von Schleimdrogen in Form von deren Extrakten zur Anwendung kommen. Erfindungsgemäß geeignete Schleimdrogen können insbesondere ausgewählt sein aus Isländischem Moos *(Lichen islandicus),* Eibisch *(Althaea officinalis L.),* Spitzwegerich *(Plantago lanceolata L.),* Malve *(Malva sylvestris L.* und *M. neglecta WALLR.),* Boxhornklee *(Trigonella foenum-graecum L.),* Salep und Quitte *(Cydonia oblonga MILL.).* Erfindungsgemäß bevorzugt sind Isländisches Moos *(Lichen islandicus)* und/oder Eibisch *(Althaea officinalis L.),* entweder allein oder in Kombination miteinander. Erfindungsgemäß ebenfalls bevorzugt ist eine Kombination von Isländischem Moos *(Lichen islandicus)* oder Eibisch *(Althaea officinalis L.)* in Form von deren jeweiligen Extrakten mit mindestens einer der anderen vorgenannten Schleimdrogen in Form von deren Extrakten. Denn diese beiden Schleimdrogen liefern im Rahmen der vorliegenden Erfindung die besten Ergebnisse.

Die einzelnen Schleimdrogen bzw. deren Extrakte sind als solche dem Fachmann hinlänglich bekannt:

Schleimdrogen werden wegen ihrer antiphlogistischen Eigenschaften äußerlich zur Behandlung von Furunkeln, Geschwüren, Drüsenschwellungen und Entzündungen des Rachenraumes, innerlich auch als Laxans, Antidiarrhoikum und zur Behandlung von Magen- und Darmentzündungen verwendet.

Schleime sind Heteropolysaccharide mit Molekulargewichten von ca. 50.000 bis 2.000.000, die durch Extraktion mit heißem oder kaltem Wasser aus der Droge gewonnen werden. Die hochviskosen Lösungen sind im Gegensatz zu den Gummen nicht klebrig. Man unterscheidet saure und neutrale Schleime. Je nach ihrer Zuckerzusammensetzung lassen sie sich in Glucomannane oder Mannane, in Galactomannane, Xylane oder Rhamnogalacturonane einteilen. Nach ihrer Lokalisierung in der Pflanze kann man Vakuolenschleime und Membranschleime unterscheiden. Als Prototyp für einen sauren Pflanzenschleim kann der Schleim der Eibischwurzel angesehen werden. Er enthält L-Rhamnose, D-Galactose, D-Galacturonsäure und D-Glucuronsäure im molaren Verhältnis von ungefähr 3 : 2 : 3 : 3. Der am einfachsten gebaute Teil des Polysaccharids besteht aus sich wiederholenden Undecasaccharid-Untereinheiten.

Isländisches Moos ist der deutsche Name für *Lichen islandicus* oder *Cetraria islandica (Parmeliaceae),* einer Flechte - entgegen dem deutschen Namen kein Moos -, die aus nördlichen Ländern, wie beispielsweise von Island, Norwegen und Schweden, exportiert wird. Isländisches Moos im eigentlichen Sinne besteht aus dem getrockneten Thallus von *Cetraria islandica* sowie dessen Zubereitungen. Die Droge enthält unter anderem Schleimstoffe und Bitterstoffe sowie bitterschmeckende Flechtensäuren. Aus der gepulverten Flechte lösen sich etwa 60 Gew.-% beim Kochen mit stark verdünnter Natriumhydrogencarbonatlösung, und beim Abkühlen der Lösung entsteht eine Gallerte. Der Extrakt besteht aus einem Polysaccharidgemisch von Lichenin und Isolichenin, einer Reihe von bitterschmeckenden Flechtensäuren (Fumarprotocetrar-, Protocetrar- und Cetrarsäure) sowie Protolichesterinsäure, die sich bei der Aufarbeitung in Lichesterinsäure umwandelt, und Usninsäure als antibiotisch wirkendem Flechtenfarbstoff. Als Schleimdroge besitzt Isländisches Moos reizlindernde Eigenschaften, es wirkt ebenso antimikrobiell. Der Bitterstoffgehalt begründet seine Anwendung bei Appetitlosigkeit. Isländisches Moos wird beispielsweise bei Katarrhen und Durchfall, als Bittertonikum, äußerlich bei schlecht heilenden Wunden, bei kosmetischen Präparaten, in Haarfixiermitteln, als Zusatz zu Biskuitmehl und dergleichen, bei Appetitlosigkeit sowie bei Schleimhautreizungen im Mund- und Rachenraum angewandt. Ein Mund- und Rachendesinfizienz auf Basis von Isländischem Moos wirkt reizlindernd bei trockenem Reizhusten. Das Kaltmazerat und andere bitterschmeckende Zubereitungen der Droge werden zur Behebung der Sub- oder Antacidität eingesetzt. Als Darreichungsformen werden je nach Anwendungsgebiet die zerkleinerte Droge für Aufgüsse sowie andere galenische Zubereitungen bzw. die zerkleinerte Droge vorzugsweise für Kaltmazerate sowie andere bitterschmeckende Zubereitungen zum Einnehmen angewandt. Für weitergehende Einzelheiten zu Isländischem Moos kann beispielsweise auf die folgende Literaturstellen verwiesen werden: Römpp Chemie-Lexikon, 9. Auflage, Band 3, Georg Thieme Verlag, Stuttgart/New York, 1990, Seiten 2055/2056, Stichwort: "Isländisches Moos"; HagerROM 2001, Springer Verlag, Heidelberg, Stichworte: "Cetraria", "Cetraria ericetorum OPIZ", "Cetraria islandica (L.) ACHARIUS" und "Lichen islandicus (Isländisches Moos)"; Monographie "Lichen islandicus (Isländisches Moos)", Bundesanzeiger Nr. 43 vom 2. März 1989; H. Wagner, "Pharmazeutische Biologie - Drogen und ihre Inhaltsstoffe", Gustav Fischer Verlag, Stuttgart/New York, 1985, Seite 281, Stichwort: "Cetrariae Lichen (= Lichen islandicus - Isländisches Moos)".

Der Eibisch *(Althaea officinalis L.),* eingesetzt in Form der Eibischwurzel, -blätter und -blüten, wächst auf salzhaltigen und feuchten Böden Mittel-, Ost- und Südosteuropas. Die im Herbst geernteten, von der holzigen Hauptwurzel, von Wurzelfasern und Rindenschichten befreiten und bei 35 °C getrockneten Wurzelzweige und Nebenwurzeln kommen geschält oder ungeschält in den Handel. Die Braunfärbung der geschälte Droge bedeutet eine Qualitätsminderung; ein nachträgliches "Schönen" mit Sulfitlösung ist unzulässig. Weiterhin kommen die vor oder während der Blüte gesammelten, etwa 10 cm langen, etwa 8 cm breiten, drei- bis fünflappigen, graufilzig behaarten getrockneten Blätter und die im Juli/August gesammelten, fleischfarbenen getrockneten Kronblätter zur Anwendung. Der Eibisch ist eine mehrjährige, etwa 1 m hohe Staude, die generativ oder vegetativ vermehrt wird. Die im Spätherbst geerntete Wurzel enthält bis zu 15 % Schleimstoffe. Im Frühjahr und Sommer liegt der Schleimgehalt bei 5 bis 6 %. Der Schleimgehalt der Blatt- und Blütendroge beträgt 6 bis 9 %. Der Schleim ist in der Wurzel in bestimmten Schleimzellen des Rinden- und Holzparenchyms lokalisiert. Er besteht aus Galacturonorhamnanen, Glucanen und Arabinogalactanen. Wäßrige Auszüge aus der Wurzel sollen durch Mazeration bei Zimmertemperatur hergestellt werden, damit die reichlich vorhandene Stärke nicht durch Quellung störend wirkt. Eibischdrogen werden für Teezubereitungen oder zur Herstellung von Sirupus Althaeae verwendet. Das Hauptanwendungsgebiet sind entzündliche Reizzustände des Rachenraums. Äußerlich kommt der Eibisch zu erweichenden Umschlägen, Bädern und Kataplasmen zur Anwendung.

Der Spitzwegerich *(Plantago lanceolata L.)* ist in ganz Europa sowie in Nord- und Mittelasien verbreitet. Die Droge stammt von wild wachsenden Pflanzen und Kulturen vor allem Südosteuropas. Die getrockneten, oliv- bis bräunlichgrün gefärbten, lanzettlichen Blattspreiten mit etwa drei bis sieben parallel verlaufenden Nerven können verwendet werden. Die Herba-Droge besteht aus den getrockneten Blättern, Stengeln und ganzen Blüten. Neben dem Schleim enthält der Spitzwegerich als weitere Inhaltsstoffe Tannine, das Iridoidglykosid Aucubin (1,9 bis 2,4 %), das für die Dunkelfärbung einer nicht sorgfältig getrockneten Droge verantwortlich ist und zur Identitätsprüfung herangezogen wird, ferner das Senföl Sulforaphen. Der Spitzwegerich, insbesondere das Spitzwegerichblätter-Kraut, findet insbesondere Anwendung in Form von Tees und Sirupen bei Entzündungen des Mund- und Rachenraums.

Die Malve *(Malva sylvestris L.* und *M. neglecta WALLR.),* insbesondere eingesetzt in Form von Malvenblüten und -blättern, ist in Europa, Kleinasien, im Mittelmeergebiet und in Vorderindien heimisch. Zur Anwendung kommen die zur Blütezeit gesammelten, rosa-violett gefärbten Blüten bzw. die durch das Trocknen stark eingeschrumpften, gefalteten Blätter, die zumeist in Paketform in den Handel kommen. Die Malve ist ein zwei- bis mehrjähriges Kraut. Der Schleimgehalt von Blüten und Blättern liegt bei 6 bis 8 %. Bei der Hydrolyse liefert der Schleim Glucose, Arabinose, Rhamnose und Galactose. Malvenblüten werden zu Gurgelwässern und Bädern und zusammen mit den Blättern in Teemischungen als Expectorans verwendet.

Der Bockshornklee *(Trigonella foenum-graecum L.),* insbesondere eingesetzt in Form des Bockshornkleesamens, ist im gesamten Mittelmeergebiet, sowie in Osteuropa, Indien und China beheimatet. Der Hauptdrogenimport stammt aus den Kulturen Indiens und Marokkos. Zur Anwendung kommen die braunrötlich gefärbten, vierseitigen und rautenförmigen, etwa 5 mm langen und etwa 3 mm breiten, flachgedrückten, sehr harten Samen, die durch eine Furche charakterisiert sind. Die Droge enthält außer fettem Öl 20 bis 30 % Schleim, Trigonellin, Nicotinsäureamid, Cholin, Bitterstoffe und Saponine. Die Herstellung des Schleims erfolgt aus dem gepulvertem Samen. Das Pulver wird angewendet äußerlich zu Umschlägen bei Furunkeln, Geschwüren und Drüsenschwellungen, innerlich als Expectorans und Roborans.

Salep bzw. Salep Tuber, insbesondere eingesetzt in Form der Salepknolle, ist von verschiedenen Orchideen-Arten abgeleitet, besonders *Orchis mascula L., Orchis morio L., Orchis militaris L., Anacamptis pyramidalis L. RICH.* und *Platanthera bifolia L. RICH.* Zur Anwendung kommen die etwa 4 cm langen bis etwa 3 cm dicken, runzeligen, harten, bräunlichen Knollen, die nach der Ernte von der Korbschicht befreit, mit siedendem Wasser abgebrüht und bei künstlicher Wärme getrocknet werden. Salepschleim, der bis zu 50 % vorwiegend aus Glucomannan bzw. Glucan besteht, wird hauptsächlich in der Kinderheilpraxis als Antidiarrhoikum verwendet.

Die Quitte *(Cydonia oblonga MILL.)* ist im südöstlichen Arabien heimisch. Hautdrogenimporte kommen aus Spanien, Portugal und Persien. Zur Anwendung kommen die reifen, getrockneten, rot-braun bis braun-violett gefärbten, etwas abgeplatteten Samen der Quittenscheinfrucht. Die Samen sind außen zum Teil mit einer eingetrockneten Schleimkruste versehen und oft miteinander verklebt. Sie besitzen einen schwachen Bittermandelgeschmack. In der Epidermis findet sich ca. 22 % Schleim, der zum größten Teil wasserlöslich ist. Die Zuckerkomponenten sind Arabinose, Xylose und Uronsäure, die zum Teil methyliert ist. Die Droge kommt nur unzerkleinert zur Anwendung. Der Quittenschleim wird äußerlich bei Lippen- und Brustwarzenrhagaden, bei Verbrennungen und Dekubitus in Salben- oder Cremeform verwendet.

Erfindungsgemäß besonders gute Ergebnisse werden mit Isländischem Moos und/oder Eibisch, entweder einzeln oder in Kombination miteinander, erzielt. Ohne sich auf eine bestimmte Theorie festlegen zu wollen, läßt sich die besonders gute Wirkung des Eibisch möglicherweise durch den sauren Pflanzenschleim erklären.

Für weitere Einzelheiten zu Schleimdrogen und ihren Zubereitungen, Wirkungen und Anwendungen kann verwiesen werden auf H. Wagner "Pharmazeutische Biologie - Drogen und ihre Inhaltsstoffe", Gustav Fischer Verlag, Stuttgart/New York, 1985, insbesondere Seiten 280 ff.

Was die erfindungsgemäß eingesetzte Schleimdroge anbelangt, so wird diese in Form eines Extraktes, insbesondere eines Trockenextraktes, zugesetzt. Ein solcher Trockenextrakt ist vorteilhafterweise auf Basis eines wäßrigen, alkoholischen oder wäßrig-alkoholischen Extraktes, bevorzugt auf Basis eines wäßrigen Extraktes, erhältlich (z. B. durch übliche Methoden, wie beispielsweise Abziehen des Wassers und/oder Alkohols im Vakuum gegebenenfalls unter Erwärmen, Lyophilisation etc.).

Das Droge/Extrakt-Verhältnis kann dabei in weiten Bereichen variieren. Erfindungsgemäß wird ein Droge/Extrakt-Verhältnis im Bereich von 5 : 1 bis 8 : 1 verwendet.

Die Verwendung eines Extraktes einer Schleimdroge bietet gegenüber der Verwendung der Droge als solcher den entscheidenden Vorteil, daß hohe Konzentrationen Schleimdroge zur Anwendung kommen, so daß eine besonders gute therapeutische Wirkung erzielt wird. Besonders herausragende therapeutische Ergebnisse werden erzielt, wenn die Schleimdroge als Extrakt, vorzugsweise als Trockenextrakt, mit einem Droge/Extrakt-Verhältnis von mindestens 1 : 1 oder mehr zugesetzt ist.

Was die Menge an Schleimdrogenextrakt(en) in der erfindungsgemäßen pharmazeutischen Zusammensetzung bzw. Kombination anbelangt, so kann die Menge in weiten Bereichen variieren. Im allgemeinen liegt die Menge an Schleimdroge(n) in Form von deren Extrakt(en) im Bereich von 0,5 bis 20 Gew.-%, insbesondere 1 bis 10 Gew.-%, bezogen auf die pharmazeutische Zusammensetzung bzw. Kombination, d. h. bezogen auf die feste Dosierungsform. Dennoch kann es anwendungsbedingt gegebenenfalls vorteilhaft oder erforderlich sein, von den vorgenannten Mengenangaben abzuweichen.

Was das erfindungsgemäß eingesetzte Lokalanästhetikum anbelangt, so wird erfindungsgemäß Benzocain verwendet.

Lokalanästhetika heben reversibel und örtlich begrenzt die Erregbarkeit der schmerzvermittelnden sensiblen Endorgane und das Leitungsvermögen der sensiblen Nervenfasern auf. Als Folge davon wird die Schmerzempfindung ohne Beeinträchtigung des Bewußtseins vorübergehend ausgeschaltet. Die Wirkung von Lokalanästhetika auf die sensorischen Nervenendigungen ist nicht spezifisch, die verschiedenen erregbaren Strukturen sind allerdings unterschiedlich empfindlich. Daß z. B. die motorischen Funktionen bei den für Lokalanästhetika üblichen Dosierungen nicht ausfallen, beruht vor allem darauf, daß die motorischen Nervenfasern einen größeren Durchmesser als die sensorischen, für die "Schmerzleitung" wichtigen Nervenfasern besitzen. Für weitergehende Einzelheiten zu Lokalanästhetika kann beispielsweise verwiesen werden auf E. Mutschler et al. "Mutschler Arzneimittelwirkungen - Lehrbuch der Pharmakologie und Toxikologie", 8. Auflage, Wissenschaftliche Verlagsgesellschaft mbH Stuttgart, 2001, Seiten 267 ff., und Römpp Chemie-Lexikon, 10. Auflage, Band 3, Georg Thieme Verlag, Stuttgart/New York, 1997, Seite 2442, Stichwort: "Lokalanästhetika", sowie die dort jeweils referierte Literatur.

Wie zuvor beschrieben, wird als Lokalanästhetikum in der erfindungsgemäßen pharmazeutischen Zusammensetzung Benzocain eingesetzt. Denn die Anmelderin hat überraschenderweise herausgefunden, daß im Rahmen der erfindungsgemäßen pharmazeutischen Zusammensetzung bzw. Kombination Benzocain zusammen mit der oder den Schleimdrogen die beste Wirkung entfaltet, Ohne sich auf eine bestimmte Theorie festlegen zu wollen, läßt sich die besonders gute Wirkung des Benzocains, eines neutralen nichtionisierbaren primären Amins, möglicherweise darauf zurückführen, daß die durch das Benzocain bewirkte Blockade der Erregungsleitung darauf beruht, daß das normale Membrangefüge durch die "Einlagerung" des Benzocains in die Lipidphase so desintegriert wird, daß es dadurch indirekt zu einer Blockade des Natriumkanals kommt, d. h. dieser Wirkmechanismus von dem anderer Lokalanästhetika des klassischen Typs (z. B. auf Basis sekundärer oder tertiärer Amine) abweicht. Dieser abweichende Wirkmechanismus ist insofern von Bedeutung, als entzündetes Gewebe infolge einer lokalen Lactatacidose einen niedrigeren pH-Wert gegenüber normalem Gewebe aufweist. Klassische Lokalanästhetika vom Typ sekundärer oder tertiärer Amine liegen dann bei einem pH-Wert von 7,4 und weniger in der Wasserphase größtenteils ionisiert vor, wodurch das Penetrationsvermögen erniedrigt wird und - im Gegensatz zu dem nichtionisierbaren Benzocain - ein Wirkungsverlust eintreten kann. Daher wird erfindungsgemäß eine pharmazeutische Kombination von mindestens einer Schleimdroge (z. B. Eibisch und/oder Isländisches Moos) in Form von deren Extrakt mit Benzocain eingesetzt.

Die Menge an Lokalanästhetikum in der pharmazeutischen Zusammensetzung kann in weiten Bereichen variieren. Im allgemeinen liegt sie im Bereich von 0,05 bis 2 Gew.-%, insbesondere 0,1 bis 1 Gew.-%, vorzugsweise 0,2 bis 0,6 Gew.-%, bezogen auf die pharmazeutische Zusammensetzung bzw. Kombination. Dennoch kann es anwendungsbedingt gegebenenfalls vorteilhaft oder erforderlich sein, von den vorgenannten Mengen abzuweichen.

Neben den vorgenannten Wirk- und Inhaltsstoffen kann die erfindungsgemäße pharmazeutische Zusammensetzung außerdem noch weitere Wirk- und/oder Inhaltsstoffe enthalten, beispielsweise Verarbeitungshilfsmittel, Aromastoffe, Geschmacksstoffe, Süßstoffe und Süßungsmittel, Säuerungsmittel, Stabilisatoren und/oder Antiseptika.

Im allgemeinen enthält die erfindungsgemäße pharmazeutische Zusammensetzung die Wirk- und/oder Inhaltsstoffe in einer festen Matrix bzw. Masse. Die Wirk- und/oder Inhaltsstoffe sind also in einer Matrix inkorporiert bzw. eingelagert, so daß sie wirksam geschützt und homogen verteilt sind.

Die feste Dosierungsform und die Inkorporierung der Wirk- und/oder Inhaltsstoffe in eine feste Matrix bzw. Masse bietet eine Reihe von Vorteilen: Zum einen lassen sich auf diese Weise die Wirk- und Inhaltsstoffe besser dosieren, d. h. es wird eine verbesserte Dosiergenauigkeit erreicht. Zum anderen wird dadurch die Applikations- bzw. Einnahmemöglichkeit verbessert, d. h. die Einnahmemöglichkeit für den Patienten ist quasi überall gegeben (z. B. auf Reisen, im Freien etc.), da keine speziellen Zubereitungsformen angemischt werden müssen. Darüber hinaus liefert die feste Dosierungsform den entscheidenden Vorteil, daß die Wirkstoffe in festen Zubereitungen im allgemeinen lagerstabiler sind. Weiterhin gewährt die feste Dosierungsform eine definierte Verweilzeit bzw. Verweildauer im Mund- und Rachenraum und somit eine effiziente Therapierung. Schließlich ermöglicht die feste Dosierungsform, insbesondere durch die Einlagerung der Wirk- und Inhaltsstoffe in die Matrix, eine verbesserte Kombination mit anderen Wirkstoffen und deren gleichmäßige, homogene Verteilung über die Matrix.

Als Matrix bzw. Masse für die Einlagerung der Wirk- und Inhaltsstoffe eignen sich insbesondere Zucker aller Art und/oder Zuckeraustauschstoffe aller Art. Beispiele für erfindungsgemäß geeignete Zucker sind beispielsweise Saccharose, Glucose, insbesondere Dextrose, und Fructose. Erfindungsgemäß geeignete Zuckeraustauschstoffe sind insbesondere ausgewählt aus Zuckeralkoholen. Erfindungsgemäß bevorzugte Zuckeraustauschstoffe sind ausgewählt aus der Gruppe von Mannit, Xylit, Sorbit, Isomalt, Maltitsirup, Lactit, Leucrose, Fructooligosacchariden, Glucanen, Polyglucose, besonders bevorzugt Isomalt.

Während Zucker und Süßstoffe gemeinsam als Süßungsmittel bezeichnet werden, werden - im Gegensatz zu den intensiv schmeckenden Süßstoffen - Zuckeraustauschstoffe technologisch wie Saccharose eingesetzt, d. h. sie besitzen einen "Körper" und einen physiologischen Brennwert ("nutritive Zukkeraustauschstoffe"). Die Süßkraft entspricht in weiten Grenzen etwa der von Saccharose. Der physiologische Vorteil der Zuckeraustauschstoffe im Vergleich zu Saccharose liegt in der insulinunhabhängigen Metabolisierung (vorteilhaft z. B. für Diabetiker) und in der zum Teil verminderten kariogenen Wirkung. Für einige Zuckeraustauschstoffe (z. B. Xylit) ist eine antikariogene Wirkung beschrieben.

Der Begriff "Zuckeralkohol" ist die Gruppenbezeichnung für die aus Monosacchariden durch Reduktion der Carbonylgruppe entstehenden Polyhydroxyverbindungen, die keine Zucker sind, gleichwohl aber süß schmecken und deshalb gegebenenfalls Verwendung als Zuckeraustauschstoffe finden können. Man unterscheidet bei diesen im allgemeinen kristallinen, wasserlöslichen Polyolen nach der Anzahl der im Molekül enthaltenen Hydroxygruppen sogenannte Tetrite, Pentite, Hexite usw. Natürlich vorkommende Zuckeralkohole sind z. B. Glycerin, Threit und Erythrit, Adonit (Ribit), Arabit (früher: Lyxit) und Xylit, Dulcit (Galactit), Mannit und Sorbit (Glucit).

Für weitergehende Einzelheiten zu den Begriffen "Zucker", "Zuckeraustauschstoffe" und "Zuckeralkohole" kann beispielsweise verwiesen werden auf Römpp Chemie-Lexikon, 10. Auflage, Band 6, Georg Thieme Verlag, Stuttgart/New York, 1999, Seiten 5096 bis 5100, Stichworte: "Zucker", "Zukkeralkohole" und "Zuckeraustauschstoffe".

Die Menge an Matrix bzw. fester Masse, insbesondere an Zuckern und/oder Zuckeraustauschstoffen, kann in weiten Bereichen variieren. Die Menge an Matrixmasse (d. h. Zuckern und/oder Zuckeraustauschstoffen) liegt im allgemeinen im Bereich von 80 bis 99,45 Gew.-%, insbesondere 85 bis 99 Gew.-%, bevorzugt 90 bis 99 Gew.-%, bezogen auf die pharmazeutische Zusammensetzung. Dennoch kann es anwendungsbedingt bzw. fallbezogen gegebenenfalls erforderlich oder vorteilhaft sein, von den vorgenannten Mengen abzuweichen.

Gemäß einer besonders bevorzugten Ausführungsform liegt die erfindungsgemäße pharmazeutische Zusammensetzung bzw. die erfindungsgemäße feste Dosierungsform als Lutschtablette, insbesondere in Form einer Hartkaramelle, vor. Wenn die erfindungsgemäße feste Dosierungsform auf Lutschtablettenbasis gebildet ist, führt dies dazu, daß die feste Dosierungsform beim Lutschen eine therapeutisch wirksame Menge der Wirk- und/oder Inhaltsstoffe freisetzt, wenn die feste Dosierungsform im Mund- und Rachenraum eines Patienten verabreicht und gelutscht wird. Besonders gute therapeutische Ergebnisse wurden bei der Behandlung von schmerzbegleitenden entzündlichen Erkrankungen des Mund- und Rachenraums mit Lutschtabletten, vorzugsweise auf Hartkaramellenbasis erzielt, die *Lichen islandicus* und/oder *Althaea officinalis L.* in Form von deren Extrakten zusammen mit Benzocain enthalten.

Bei der bevorzugten Ausgestaltung der erfindungsgemäßen pharmazeutischen Zusammensetzung in Form einer Lutschtablette, insbesondere auf Hartkaramellenbasis, kann das Gewicht der Lutschtablette in weiten Bereichen variieren. Im allgemeinen beträgt das Gesamtgewicht der Lutschtablette 1 bis 5 g, vorzugsweise 2 bis 3 g, besonders bevorzugt 2,4 bis 2,7 g, wobei jede Lutschtablette 13 bis 260 mg, insbesondere 26 bis 130 mg, Schleimdrogenextrakt(e) (z. B. *Lichen islandicus* und/oder *Althaea officinalis L.)* zusammen mit 1,3 bis 52 mg, insbesondere 2,6 bis 26 mg, vorzugsweise 5,2 bis 15 mg, Benzocain, in einer Masse bzw. Matrix (insbesondere Zucker und/oder Zuckerersatzstoffe) von 1,9 bis 4,9 g, insbesondere 2 bis 2,6 g, enthält. Wie zuvor ausgeführt, kann die erfindungsgemäße Lutschtablette darüber hinaus noch weitere Wirk-und/oder Inhaltsstoffe enthalten, so z. B. weitere Schleimdrogen in Form von deren Extrakten, Verarbeitungshilfsmittel, Aromastoffe, Geschmacksstoffe, Süßstoffe und Süßungsmittel, Säuerungsmittel, Stabilisatoren und/oder Antiseptika. Diesbezüglich kann auf obige Ausführungen verwiesen werden.

Die Herstellung der erfindungsgemäßen pharmazeutischen Zusammensetzung erfolgt in an sich bekannter Weise. Für weitergehende Einzelheiten kann auf die nachfolgenden Ausführungsbeispiele verwiesen werden. Bei der Herstellung von Lutschtabletten auf Hartkaramellenbasis kann beispielsweise derart vorgegangen werden, daß zunächst die Wirk- und Inhaltsstoffe - gegebenenfalls nach Zerkleinerung - eingewogen werden und dann in die Grundsubstanz auf Basis von Zuckern oder Zuckerersatzstoffen (z. B. Isomalt) eingemischt werden, gefolgt von einer Erwärmung der Grundsubstanz, Formung von Lutschtabletten und nachfolgendem Abkühlen. Derartige Herstellverfahren sind dem Fachmann als solche wohlbekannt, so daß hier nicht näher darauf eingegangen zu werden braucht.

Ein weiterer Gegenstand der vorliegenden Erfindung ist die Verwendung der zuvor beschriebenen pharmazeutischen Zusammensetzung nach der vorliegenden Erfindung zur topischen Behandlung von insbesondere schmerzbegleiteten entzündlichen Erkrankungen des Mund-/Rachenraums bzw. zur Herstellung eines Arzneimittels zur topischen Behandlung von insbesondere schmerzbegleiteten entzündlichen Erkrankungen des Mund-/Rachenraums.

Für weitergehende Einzelheiten in bezug auf die erfindungsgemäße Verwendung kann auf obige Ausführungen zu der erfindungsgemäßen pharmazeutischen Zusammensetzung bzw. Kombination verwiesen werden, die in bezug auf die erfindungsgemäße Verwendung entsprechend gelten.

Die folgenden Ausführungsbeispiele dienen lediglich der Veranschaulichung der vorliegenden Erfindung, ohne sie jedoch hierauf zu beschränken.

### Ausführungsbeispiele:

### 1. Herstellungsbeispiele:

### Herstellung erfindungsgemäßer pharmazeutischer Zusammensetzungen in Form von Lutschtabletten (Hartkaramellenbasis) auf der Basis eines Extraktes von verschiedenen Schleimdrogen (Lichen islandicus, Althaea officinalis L. sowie Lichen islandicus plus Althaea officinalis L.) in Kombination mit variablen Benzocainmengen

Es werden verschiedene pharmazeutische Zusammensetzungen in Form von Lutschtabletten auf Hartkaramellenbasis hergestellt. Für die zuckerhaltige Variante können Fructose- und/oder Glucosesirup eingesetzt werden, während für die zuckerfreie Variante Zuckeraustauschstoffe, insbesondere Zuckeralkohole, vorzugsweise Maltit oder Isomaltit (Palatinit^{®}), als Lutschtablettenbasis bzw. Hartkaramellenbasis (Matrix) eingesetzt werden können, wobei sowohl bei der zuckerhaltigen als auch bei der zuckerfreien Variante gegebenenfalls variable Mengen an Süßstoffen (z. B. Acesulfam, Aspartam, Cyclamat, Saccharin, Thaumatin oder Neohesperidin) zugesetzt sein können. In die Matrix sind die entsprechenden Wirk- und Inhaltsstoffe eingearbeitet. Die Herstellung der Lutschtabletten bzw. Hartkaramellen erfolgt in an sich bekannter Weise.

Die Ausgangsstoffe werden hierzu entsprechend der jeweiligen Rezeptur eingewogen und einzeln in die Grundsubstanz/Hartkaramellenbasis (z. B. Isomaltit) eingemischt. Die Zugabe von Wasser kann entfallen. Der Mischvorgang erfolgt in der Wiegestation mit den entsprechenden freigegebenen kalibrierten Einwaagen. Die Rohsubstanzen werden erwärmt und über die Produktionsschiene bearbeitet und in kleine Mengen unterteilt. Zu diesen Mengen werden dann die Ausgangsstoffe bzw. Zwischenprodukte, insbesondere
- Isländisches Moos *(Lichen islandicus)* und/oder Eibisch *(Althaea officinalis L.)*
- Benzocain (Lokalanästhetikum)
- gegebenenfalls Kräuteraromen
- gegebenenfalls Pfefferminzöl und
- gegebenenfalls Weinsäure (Stabilisator/Säuerungsmittel)
über eine separate Dosiereinheit zugegeben.

Es erfolgt ein erneutes Mischen und Vermischen der gesamten Charge. Die Menge wird in einem Zwischenlager zwischengelagert und auf die Dosiennaschine oder Dosiereinheit zurückgeführt. Dort werden die Lutschtabletten in ihrer entsprechenden Größe hergestellt und während des Prozesses verwogen. Die fertigen Tabletten werden sofort zu der Blisterstation weitergeleitet und mit freien Packmitteln verpackt. Das anschließende Palettieren erfolgt am Ende des Blistervorgangs. Die Produkte kommen in die Quarantäne und werden von der Qualitätskontrolle auf die entsprechenden Wirk- bzw. Inhaltsstoffe und das Gewicht überprüft und nach den entsprechenden Prüfungen freigegeben. Auf diese Weise werden beispielsweise zuckerfreie Lutschtabletten ("Hartkaramellen") mit wechselnden Mengen an Lokalanästhetikum (Benzocain) (0,2 bis 0,6 Gew.-%, bezogen auf das Lutschtablettengewicht) hergestellt, wie in den nachfolgenden Tabellen dargestellt (erste Serie):

**Tabelle 1: Isländisches Moos (Lichen islandicus) mit 0,2 Gew.-% Benzocain (Lutschtabletten zuckerfrei)**

| **Rohstoffe** | **Menge trocken/Lutschtablette mg** | **Menge trocken/1.000 kg** |
|---|---|---|
| Palatinit^{®} M (Isomalt) | 2.503,300 | 962,808 |
| Wasser | 0,000 | 0,000 |
| *Lichen islandicus* (Extrakt, Droge/Extrakt-Verhältnis 7:1) | 50,000 | 19,231 |
| Kräuteraroma | 26,000 | 10,000 |
| Pfefferminzöl | 0,500 | 0,192 |
| Benzocain | 5,200 | 2,000 |
| Weinsäure | 15,000 | 5,769 |
| gesamt | 2.600,000 | 1.000,000 |

**Tabelle 2: Isländisches Moos (Lichen islandicus) mit 0,3 Gew.-% Benzocain (Lutschtabletten zuckeirfrei)**

| **Rohstoffe** | **Menge trocken/Lutschtablette mg** | **Menge trocken/1.000 kg** |
|---|---|---|
| Palatinit^{®} M (Isomalt) | 2.500,700 | 961,808 |
| Wasser | 0,000 | 0,000 |
| *Lichen islandicus* (Extrakt, Droge/Extrakt-Verhältnis 7:1) | 50,000 | 19,231 |
| Kräuteraroma | 26,000 | 10,000 |
| Pfefferminzöl | 0,500 | 0,192 |
| Benzocain | 7,800 | 3,000 |
| Weinsäure | 15,000 | 5,769 |
| gesamt | 2.600,000 | 1.000,000 |

**Tabelle 3: Isländisches Moos (Lichen islandicus) mit 0,4 Gew.-% Benzocain (Lutschtabletten zuckerfrei)**

| **Rohstoffe** | **Menge trocken/Lutschtablette mg** | **Menge trocken/1.000 kg** |
|---|---|---|
| Palatinit^{®} M (Isomalt) | 2.498,100 | 960,808 |
| Wasser | 0,000 | 0,000 |
| *Lichen islandicus* (Extrakt, Droge/Extrakt-Verhältnis 7:1) | 50,000 | 19,231 |
| Kräuteraroma | 26,000 | 10,000 |
| Pfefferminzöl | 0,500 | 0,192 |
| Benzocain | 10,400 | 4,000 |
| Weinsäure | 15,000 | 5,769 |
| gesamt | 2.600,000 | 1.000,000 |

**Tabelle 4: Isländisches Moos (Lichen islandicus) mit 0,5 Gew.-% Benzocain (Lutschtabletten zuckerfrei)**

| **Rohstoffe** | **Menge trocken/Lutschtablette mg** | **Menge trocken/1.000 kg** |
|---|---|---|
| Palatinit^{®} M (Isomalt) | 2.495,500 | 959,808 |
| Wasser | 0,000 | 0,000 |
| *Lichen islandicus* (Extrakt, Droge/Extrakt-Verhältnis 7:1) | 50,000 | 19,231 |
| Kräuteraroma | 26,000 | 10,000 |
| pfefferminzöl | 0,500 | 0,192 |
| Benzocain | 13,000 | 5,000 |
| Weinsäure | 15,000 | 5,769 |
| gesamt | 2.600,000 | 1.000,000 |

**Tabelle 5: Isländisches Moos (Lichen islandicus) mit 0,6 Gew.-% Benzocain (Lutschtabletten zuckerfrei)**

| **Rohstoffe** | **Menge trocken/Lutschtablette** mg | **Menge trocken/1.000 kg** |
|---|---|---|
| Palatinit^{®} M (Isomalt) | 2-492,900 | 958,808 |
| Wasser | 0,000 | 0,000 |
| *Lichen islandicus* (Extrakt, Droge/Extrakt-Verhältnis 7:1) | 50,000 | 19,231 |
| Kräuteraroma | 26,000 | 10,000 |
| Pfefferminzöl | 0,500 | 0,192 |
| Benzocain | 15,600 | 6,000 |
| Weinsäure | 15,000 | 5,769 |
| gesamt | 2.600,000 | 1.000,000 |

Auf analoge Art und Weise wie die erste Serie von Lutschtabletten mit einem Extrakt von *Lichen islandicus* wurden eine zweite Serie von Lutschtabletten mit einem Extrakt von *Althaea officinalis L.* und eine dritte Serie von Lutschtabletten mit einem Gemisch (1 : 1) von Extrakten von *Lichen islandicus* und *Althaea officinalis L.,* jeweils mit variablen Benzocaingehalten zwischen 0,2 und 0,6 Gew.-%, hergestellt.

### 2. Anwendungsbeispiele:

Zwanzig Patienten mit akuten schmerzhaften Halsentzündungen infolge von Erkältungserkrankungen, einhergehend mit schmerzhafte Schluckbeschwerden, wurden ohne zusätzliche systemische Therapie ausschließlich topisch mittels Lutschtabletten der ersten Serie mit Isländischem Moos *(Lichen islandicus)* therapiert, Bei zehn Patienten wurde eine erfindungsgemäße, feste pharmazeutische Zusammensetzung auf Hartkaramellenbasis mit 0,5 Gew.-% Benzocain, wie im vorhergehenden Herstellungsbeispiel (Tabelle 4) beschrieben, eingesetzt, während bei den übrigen zehn Patienten ein handelsübliches Präparat nur mit Isländischem Moos (Lutsch-/Kaupastillen mit Gummi arabicum als Matrix) in geringerer Dosierung (80 mg wäßriger Auszug aus Isländischem Moos mit Droge/Extrakt-Verhältnis 0,4 - 0,8 : 1 pro Pastille) und ohne Lokalanästhetikum zur Anwendung kam.

Bei beiden Präparaten zeigte sich eine entzündungshemmende Wirkung. Bei der mit der erfindungsgemäßen pharmazeutischen Zusammensetzung therapierten Patienten trat jedoch aufgrund des Vorhandenseins des Lokalanästhetikums unmittelbar nach der Anwendung (Lutschen) eine deutliche Linderung der Schmerzen auf, während die schmerzhaften Schluckbeschwerden bei dem nichterfindungsgemäßen Präparat zunächst bestehen blieben. Aufgrund der höheren Dosierung und der effizienteren Formulierung bzw. Galenik (Isomaltitmatrix) trat bei den mit der erfindungsgemäßen Zusammensetzung behandelten Patienten bereits nach ein bis zwei Tagen eine deutliche Linderung der Entzündungen auf und waren die Entzündungen bereits innerhalb von fünf bis acht Tagen vollständig abgeklungen, während dies bei dem nicht erfindungsgemäßen Präparat mit geringerer Dosierung an Isländischem Moos und ohne Lokalanästhetikum deutlich länger andauerte: Bei der Behandlung mit der nichterfindungsgemäßen Zusammensetzung traten erste Linderungen erst nach etwa drei bis vier Tagen auf und waren die letzten Symptome erst nach mehr als zehn Tagen Behandlungsdauer verschwunden.

Die Untersuchungen zeigen, daß die Anwendung der erfindungsgemäßen Zusammensetzung in Form einer binären Kombination gegenüber der nichterfindungsgemäßen Anwendungsform einen deutlich gesteigerten Therapieerfolg bewirkt, da durch die erfindungsgemäße Zusammensetzung zum einen die Schmerzsymptomatik deutlich gelindert werden kann und zum anderen aufgrund der höheren Dosierung mit dem stärkeren Droge/Extrakt-Verhältnis ein schnellerer Therapieerfolg bewirkt wird.

Vergleichbare Ergebnisse wurden mit den Lutschtabletten der zweiten und dritten Serie erreicht.

Die erfindungsgemäße pharmazeutische Zusammensetzung führt bei der Behandlung von akuten schmerzhaften Halsentzündungen zu einer eindrucksvollen Besserung der hiermit einhergehenden Symptome, und zwar sowohl im Hinblick auf die Schmerz- als auch auf die Entzündungssymptomatik. Hierin liegt eine entscheidender Vorteil der erfindungsgemäßen Kombination.

## Patentansprüche

1. Pharmazeutische Zusammensetzung in Form einer festen Dosierung zur topischen Behandlung von entzündlichen Erkrankungen des Mund- und Rachenraums, enthaltend in Kombination
• mindestens eine Schleimdroge, ausgewählt aus Isländischem Moos *(Lichen islandicus),* Eibisch *(Althaea officinalis L.),* Spitzwegerich *(Plantago lanceolata L*.), Malve *(Malva sylvestris L.* und *M*. *neglecta WALLR.*), Boxhornklee *(Trigonella foenum-graecum L.*), Salep und Quitte (*Cydonia oblonga MILL.),* wobei die Schleimdroge in Form eines Extraktes mit einem Droge/Extrakt-Verhältnis im Bereich von 5 : 1 bis 8 : 1 in einer Menge von 0,5 bis 20 Gew.-%, bezogen auf die pharmazeutische Zusammensetzung, zugesetzt ist; und
• 0,05 bis 2 Gew.-%, bezogen auf die pharmazeutische Zusammensetzung, mindestens eines Lokalanästhetikums, wobei als Lokalanästhetikum Benzocain verwendet wird.

2. Pharmazeutische Zusammensetzung nach Anspruch 1, wobei die Schleimdroge in Form eines Trockenextraktes zugesetzt ist.

3. Pharmazeutische Zusammensetzung nach Anspruch 1 oder 2, enthaltend den Schleimdrogenextrakt in Mengen von 1 bis 10 Gew.-%, bezogen auf die pharmazeutische Zusammensetzung.

4. Pharmazeutische Zusammensetzung nach einem oder mehreren der vorangehenden Ansprüche, wobei die Schleimdroge ausgewählt ist aus Isländischem Moos *(Lichen islandicus)* und/oder Eibisch *(Althaea officinalis L.).*

5. Pharmazeutische Zusammensetzung nach einem oder mehreren der vorangehenden Ansprüche, enthaltend das Lokalanästhetikum in Mengen von 0,1 bis 1 Gew.-%, vorzugsweise 0,2 bis 0,6 Gew.-%, bezogen auf die pharmazeutische Zusammensetzung.

6. Pharmazeutische Zusammensetzung nach einem oder mehreren der vorangehenden Ansprüche, enthaltend die Wirk- und/oder Inhaltsstoffe in einer festen Matrix und/oder Masse, insbesondere in einer Matrix und/oder Masse auf Basis von Zuckern und/oder Zuckeraustauschstoffen, insbesondere wobei die Zucker ausgewählt sind aus der Gruppe von Saccharose, Glucose, insbesondere Dextrose, und Fructose und/oder insbesondere wobei die Zuckeraustauschstoffe ausgewählt sind aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose, besonders bevorzugt Isomalt, und/oder insbesondere wobei der Zucker und/oder die Zuckeraustauschstoffe in Mengen von 80 bis 99,45 Gew.-%, insbesondere 85 bis 99 Gew.-%, bevorzugt 90 bis 99 Gew.-%, bezogen auf die pharmazeutische Zusammensetzung, vorhanden ist/sind.

7. Pharmazeutische Zusammensetzung nach einem oder mehreren der vorangehenden Ansprüche, wobei die feste Dosierungsform eine Lutschtablette ist, insbesondere in Form einer Hartkaramelle, und/oder wobei die feste Dosierungsform auf Lutschtablettenbasis gebildet ist, so daß die feste Dosierungsform eine therapeutisch wirksame Menge der Wirk- und/oder Inhaltsstoffe beim Lutschen freisetzt, wenn die feste Dosierungsform im Mund- und Rachenraum eines Patienten verabreicht und gelutscht wird und/oder wobei die feste Dosierungsform den Schleimdrogenextrakt, insbesondere von *Lichen islandicus* und/oder *Althaea ficinalis L.,* zusammen mit Benzocain enthält.

8. Verwendung einer pharmazeutischen Zusammensetzung, wie in einem oder mehreren der vorangehenden Ansprüche definiert, zur Herstellung eines Arzneimittels zur topischen Behandlung von insbesondere schmerzbegleiteten entzündlichen Erkrankungen des Mund- und Rachenraums.

## Claims

1. Pharmaceutical composition in the form of a solid dose for the topical treatment of inflammatory diseases of the oral cavity and pharyngeal cavity, comprising in combination
• at least one mucilaginous drug, selected from Icelandic moss *(Lichen islandicus),* marsh mallow *(Althaea officinalis L.),* ribwort *(Plantago lanceolata L.),* mallow *(Malva silvestris L.* and *M. neglecta WALLR.),* fenugreek *(Trigonella foenum-graecum L.),* salep and quince *(Cydonia oblonga MILL.),* where the mucilaginous drug is added in the form of an extract having a drug/extract ratio in the range from 5:1 to 8:1 in an amount of 0.5 to 20% by weight, based on the pharmaceutical composition; and
• 0.05 to 2% by weight, based on the pharmaceutical composition, of at least one local anaesthetic, where the local anaesthetic used is benzocaine.

2. Pharmaceutical composition according to Claim 1, where the mucilaginous drug is added in the form of a dry extract.

3. Pharmaceutical composition according to Claim 1 or 2, comprising the mucilaginous drug extract in amounts from 1 to 10% by weight, based on the pharmaceutical composition.

4. Pharmaceutical composition according to one or more of the preceding claims, where the mucilaginous drug is selected from Icelandic moss (*Lichen islandicus*) and/or marsh mallow *(Althaea officinalis L.).*

5. Pharmaceutical composition according to one or more of the preceding claims, comprising the local anaesthetic in amounts from 0.1 to 1% by weight, preferably 0.2 to 0.6% by weight, based on the pharmaceutical composition.

6. Pharmaceutical composition according to one or more of the preceding claims, comprising the active substances and/or ingredients in a solid matrix and/or composition, in particular in a matrix and/or composition based on sugars and/or sugar substitutes, in particular where the sugars are selected from the group consisting of sucrose, glucose, in particular dextrose, and fructose and/or in particular where the sugar substitutes are selected from sugar alcohols, preferably from the group consisting of mannitol, xylitol, sorbitol, isomalt, maltitol syrup, lactitol, leucrose, fructooligosaccharides, glucans, polyglucose, particularly preferably isomalt, and/or in particular where the sugar and/or the sugar substitutes is/are present in amounts from 80 to 99.45% by weight, in particular 85 to 99% by weight, preferably 90 to 99% by weight, based on the pharmaceutical composition.

7. Pharmaceutical composition according to one or more of the preceding claims, where the solid dose form is a lozenge, in particular in the form of a hard caramel, and/or where the solid dose form is formed on a lozenge basis, such that the solid dose form releases a therapeutically efficacious amount of the active substances and/or ingredients on sucking when the solid dose form is administered to and is sucked in the oral cavity and pharyngeal cavity of a patient and/or where the solid dose form contains the mucilaginous drug extract, in particular of *Lichen islandicus* and/or *Althaea officinalis L.,* together with benzocaine.

8. Use of a pharmaceutical composition, as defined in one or more of the preceding claims, for the production of a medicament for the topical treatment of, in particular, inflammatory diseases of the oral cavity and pharyngeal cavity accompanied by pain.

## Revendications

1. Composition pharmaceutique sous forme d'une forme galénique solide destinée au traitement topique de maladies inflammatoires de la cavité buccale et pharyngienne, contenant une association
• d'au moins un mucilage choisi parmi la mousse d'Islande *(Lichen islandicus),* la guimauve officinale *(Althaea officinalis L.),* le plantain lancéolé *(Plantago lanceolata L.),* la mauve sylvestre *(Malva sylvestris L.* et *M. neglecta WALLR.),* le fenugrec *(Trigonella foenum-graecum),* le bulbe d'orchidée salep et le coing *(Cydonia oblonga MILL.),* le mucilage étant ajouté sous forme d'un extrait en un rapport mucilage/extrait dans la plage allant de 5:1 à 8: 1, en une quantité de 0,5 à 20 % en poids, par rapport à la composition pharmaceutique ; et
• de 0,05 à 2 % en poids, par rapport à la composition pharmaceutique, d'au moins un anesthésique local, la benzocaïne étant utilisée en tant qu'anesthésique local.

2. Composition pharmaceutique selon la revendication 1, dans laquelle le mucilage est ajouté sous forme d'un extrait sec.

3. Composition pharmaceutique selon la revendication 1 ou 2, contenant l'extrait de mucilage en quantités de 1 à 10 % en poids, par rapport à la composition pharmaceutique.

4. Composition pharmaceutique selon une ou plusieurs des revendications précédentes, dans laquelle le mucilage est choisi parmi la mousse d'Islande *(Lichen islandicus)* et/ou la guimauve officinale *(Althaea officinalis L.*)*.*

5. Composition pharmaceutique selon une ou plusieurs des revendications précédentes, contenant l'anesthésique local en quantités de 0,1 à 1 % en poids, de préférence de 0,2 à 0,6 % en poids, par rapport à la composition pharmaceutique.

6. Composition pharmaceutique selon une ou plusieurs des revendications précédentes, contenant les principes actifs et/ou les composants dans une masse et/ou matrice solide, en particulier dans une masse et/ou matrice à base de sucres et/ou de succédanés de sucres, en particulier les sucres étant choisis dans le groupe constitué par le saccharose, le glucose, en particulier le D-glucose, et le fructose et/ou en particulier les succédanés de sucres étant choisis parmi des alcools dérivés de sucres, de préférence dans le groupe constitué par le mannitol, le xylitol, le sorbitol, l'isomalt, le sirop de malt, le lactitol, le leucrose, les fructooligosaccharides, les glucanes, le polyglucose, de façon particulièrement préférée l'isomalt, et/ou en particulier le sucre et/ou les succédanés de sucre étant présent(s) en quantités de 80 à 99,45 % en poids, en particulier de 85 à 99 % en poids, de préférence de 90 à 99 % en poids, par rapport à la composition pharmaceutique.

7. Composition pharmaceutique selon une ou plusieurs des revendications précédentes, dans laquelle la forme galénique solide est une pastille, en particulier sous forme d'un caramel dur, et/ou dans laquelle la forme galénique solide est constituée à base de pastille, de sorte que la forme galénique solide libère quand elle est sucée une quantité thérapeutiquement efficace des principes actifs et/ou composants, lorsque la forme galénique solide est administrée et sucée dans la cavité buccale et pharyngienne d'un patient et/ou dans laquelle la forme galénique solide contient l'extrait de mucilage, en particulier de *Lichen islandicus* et/ou *Althaea officinalis L.,* conjointement avec de la benzocaïne.

8. Utilisation d'une composition pharmaceutique, telle que définie dans une ou plusieurs des revendications précédentes, pour la fabrication d'un médicament destiné au traitement topique de maladies inflammatoires de la cavité buccale et pharyngienne en particulier s'accompagnant de douleur.
